(19) Europäisches Patentamt
European Patent Office
Office européen des brevets

(11) Veröffentlichungsnummer: **0 087 676**
**B1**

# (12) EUROPÄISCHE PATENTSCHRIFT

(45) Veröffentlichungstag der Patentschrift: **16.12.87**

(51) Int. Cl.⁴: **C 07 C 103/375**

(21) Anmeldenummer: **83101450.1**

(22) Anmeldetag: **16.02.83**

## (54) Verfahren zur Herstellung von o-Acylaminomethylbenzylhalogeniden.

(30) Priorität: **25.02.82 DE 3206660**

(43) Veröffentlichungstag der Anmeldung: **07.09.83 Patentblatt 83/36**

(45) Bekanntmachung des Hinweises auf die Patenterteilung: **16.12.87 Patentblatt 87/51**

(84) Benannte Vertragsstaaten: **AT BE CH DE FR GB IT LI NL SE**

(56) Entgegenhaltungen:
**EP-A-0 013 411**
**DE-A-2 834 312**
**DE-A-2 945 452**
**US-A-4 311 858**

**Houben-Weyl, Methoden der organischen Chemie", Band XI/1 (1957), S. 927**

(73) Patentinhaber: **BASF Aktiengesellschaft, Carl-Bosch- Strasse 38, D-6700 Ludwigshafen (DE)**

(72) Erfinder: **Mueller, Josef, Dr., Karl.Otto- Braun-Strasse 16, D-6700 Ludwigshafen (DE)**
Erfinder: **Wiersdorff, Walter- Wielant, Dr., Blockfeldstrasse 15, D-6704 Mutterstadt (DE)**
Erfinder: **Kirschenlohr, Werner, Dr., Alwin-Mittasch- Platz 10, D-6700 Ludwigshafen (DE)**
Erfinder: **Schwantje, Gerd, Dr., Theodor- Heuss-Strasse 2, D-6706 Wachenheim (DE)**

**EP 0 087 676 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft ein neues Verfahren zur Herstellung von o-Acylaminomethylbenzylhalogeniden der allgemeinen Formel I

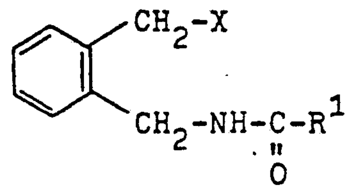

(I),

wobei X für ein Halogenatom und $R^1$ fur einen aliphatischen, cycloaliphatischen, araliphatischen oder aromatischen Rest steht.

Verbindungen des Typs I und ihre Herstellung sind bereits von Braun und Reich, Annalen, 445. 240 (1925) sowie von J. Haginiwa et al., J.Pharm.Soc.Japan, 79 (12) 1578 bis 1581 (1959) beschrieben worden.

So läßt sich beispielsweise o-Aminomethylbenzylethylether mit einer konzentrierten, wäßrigen Halogenwasserstoffsäure nur unter Druck und bei hoher Temperatur umsetzen, um den Etherrest gegen Chlor bzw. Brom auszutauschen. Es konnten dabei jedoch keine reinen Produkte isoliert werden.

Erst durch die Umsetzung mit gasförmigem Chlorwasserstoff in alkoholischer Lösung war es möglich, die gewünschte Chlorverbindung als Hydrochlorid zu isolieren. Nach Freisetzen der (wohl wegen der Ringschlußneigung) sehr empfindlichen chlorierten Base hat man dann den Acylrest in Form des Benzoylrestes eingeführt:

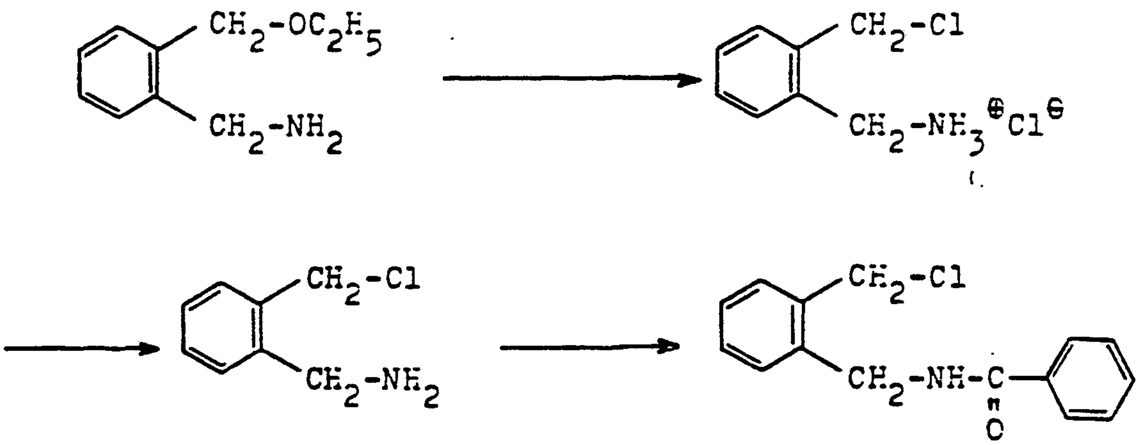

In der US-PS 4 311 858 wird die Spaltung von N-(Alkoxymethyl)-acylaminen A mit 37 %iger Salzsäure bei Raumtemperatur beschrieben. Es werden die entsprechenden N-(Chlormethyl)-acylamine B isoliert

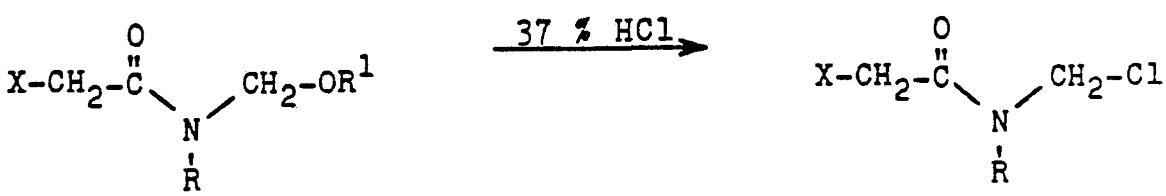


A B

Die weitere Umsetzung von N-Acylaminen unter Erhalt der N-Acylstruktur ist auch allgemein bekannt, vgl. Houben-Weyl, Methoden der organischen Chemie, 4. Auflage, Bd. 11/1, Seiten 927 und 1005 ff.. Die N-Acylamin-Struktur hat dabei den Charakter einer Schutzfunktion für das zugrundeliegende Amin; dies setzt einerseits voraus, daß die Schutzgruppe unter Rückbildung der Aminstruktur wieder abspaltbar ist und andererseits, daß sie gerade unter den Umsetzungsbedingungen, gegen die sie das Amin schützen soll, selbst stabil ist. Typische Umsetzungen dieser Art sind z. B. Oxidationen, etwa mit $KMnO_4$ oder $K_2Cr_2O_7$, Halogenierung, Sulfonierung; dabei herrschen typischerweise keine Bedingungen, unter denen eine Verseifung der Säureamidstruktur zu erwarten ist.

Die vorgenannte Umsetzung bildet insoweit keine Ausnahme, denn nach der US-A 4 311 858 findet der in Frage stehende Austausch des Etherrestes gegen Chlor mit 37 %iger Salzsäure praktisch bei Raumtemperatur

statt. Die Angabe, daß diese Umsetzung bei einer Temperatur von bis zu 100°C möglich sei, ist indessen rein spekulativ, worauf schon hindeutet, daß sie bei einer Temperatur vorgenommen werden soll, die "minimale Hydrolyse bewirkt".

Die nachstehend beschriebene Umsetzung ist, anders als die der US-A- 4 311 858, bei Raumtemperatur nicht durchführbar.

Überraschenderweise verläuft sie jedoch bei einer höheren Temperatur, nämlich bis zu 80°C, wobei darauf hinzuweisen ist, daß die Spaltung von Benzylethern nach der US-PS 2 100 822 erst oberhalb 100°C mit konzentrierter Salzsäure gelingt. Unter diesen Bedingungen werden jedoch Amide glatt gespalten (Houben-Weyl 11/1, S. 927). Überraschend war es daher, Benzylether erfindungsgemäß mit wäßriger Salzsäure zu spalten, ohne daß das Amid hydrolysiert wird.

Es wurde gefunden, daß man eine Verbindung der allgemeinen Formel (I) dadurch erhält, daß man einen o-Aminoethylbenzylalkylether der allgemeinen Formel (II)

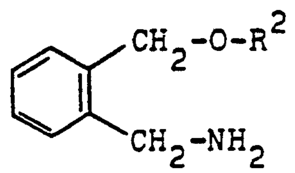

(II),

in der $R^2$ für einen beliebigen aliphatischen, insbesondere 1 bis 4 C-Atome aufweisenden Rest steht, zu dem entsprechenden Acylaminomethylbenzylether der allgemeinen Formel (III)

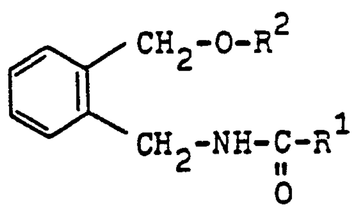

(III)

umsetzt und diese dann bei einer Temperatur von bis zu 80°C mit wäßriger Halogenwasserstoffsäure in die gewünschten Halogenverbindungen (I) überführt.

Als Rest $R^1$ sind z. B. Alkylreste mit 1 bis 4 C-Atomen oder ein Phenylrest wirtschaftlich interessant, da dieser Rest bei der Weiterverarbeitung wieder verlorengeht.

Die Verbindungen der allgemeinen Formel (I) sind interessante Zwischenprodukte für den Pflanzenschutz- und pharmazeutischen Bereich. Sie können beispielsweise als Vorstufe für die Herstellung von o-Aminomethylphenylessigsäure benutzt werden. o-Aminomethylphenylessigsäure wiederum ist ein Baustein des halbsynthetischen Antibiotikums Ceforanid:

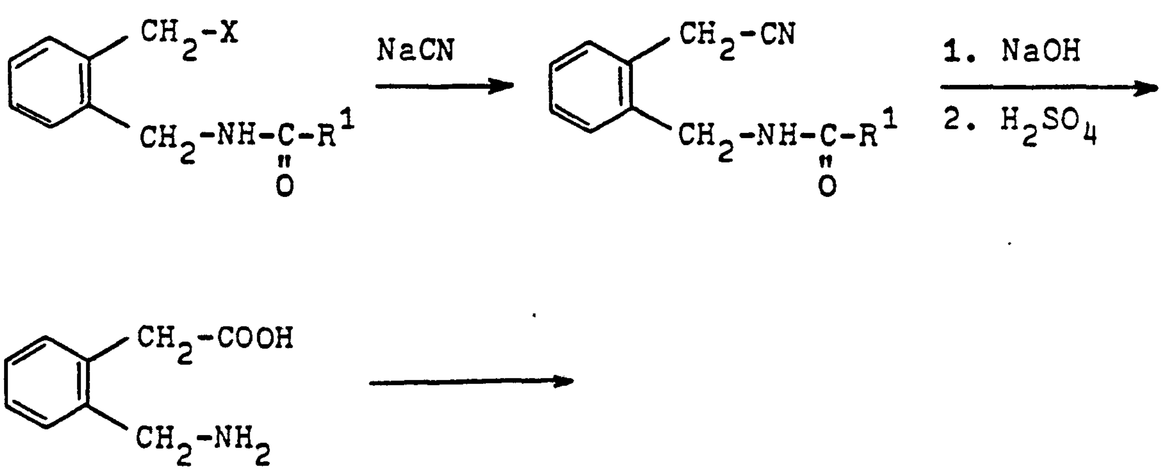

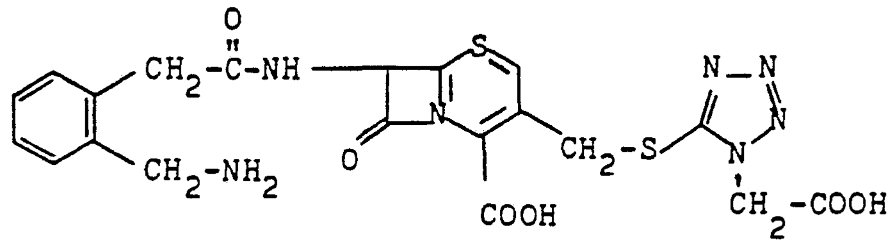

Die Umsetzung des o-Aminomethylbenzylalkylethers (II) kann in an sich bekannter Weise mit Carbonsäurechloriden oder -anhydriden in Anwesenheit oder Abwesenheit von Lösungsmitteln durchgeführt werden. Als Etherrest $R^2$ ist aus praktischen Gründen die Methylgruppe bevorzugt; jede andere greifbare Verbindung mit einem anderen Etherrest kann ebensogut verwendet werden.

Als Lösungsmittel kommt Toluol, Xylol oder ein chlorierter Kohlenwasserstoff, wie Methylenchlorid in Frage. Die Umsetzung mit Carbonsäurehalogenid wird mit Hilfe von z. B. Triethylamin oder Soda vervollständigt; eine geeignete Temperatur liegt im Bereich von 0 bis zu 200°C. Es ist möglich, bei ca. 50°C unter Kühlung das Acylierungsmittel zum vorgelegten Amin zuzusetzen oder umgekehrt. Nach Zusatz von Wasser und Abtrennung der wäßrigen Phase, kann die Acylverbindung unmittelbar halogeniert oder nach Entfernen des Lösungsmittels zunächst kristallisiert werden.

Der Umsatz zur Halogenverbindung geschieht durch Verwendung von wäßriger Halogenwasserstoffsäure, d.h. HCl, HBr, gegebenenfalls auch HJ. Als besonders einfach hat sich die Umsetzung des Benzylethers mit wäßriger Salzsäure erwiesen. Der Gehalt an HCl kann z. B. 5 bis 40 Gew.% betragen; vorzugsweise arbeitet man mit überschüssiger, handelsülicher, z. B. 36 %iger Salzsäure. Die Umsetzung kann absatzweise oder fortlaufend stattfinden und zwar vorteilhaft in Gegenwart eines weiteren Lösungsmittels, wie z. B. Methylenchlorid, Chloroform, Dichlorethan, 1,2-Dichlorpropan oder Toluol oder Chlorbenzol. Das Lösungsmittel sollte HCl ausreichend lösen. Die Umsetzung gelingt aber auch in Abwesenheit von Lösungsmitteln.

Die Umsetzung erfolgt bei einer Temperatur von bis zu 80°C, insbesonderer bis zu 60°C.

Die beiden nacheinander ablaufenden Umsetzungen werden vorteilhaft im gleichen Lösungsmittel vorgenommen, wobei auf eine Isolierung der Zwischenstufe verzichtet wird ("Eintopfreaktion"). In der Möglichkeit zu dieser Verfahrensvereinfachung besteht ein wesentlicher Vorteil der Erfindung gegenüber dem bekannten Weg.

Unter den erfindungsgemäßen Bedingungen fallen die gewünschten Chlorverbindungen I in einer hohen Reinheit an; nach Neutralisation und Abtrennen der wäßrigen Phase kristallisieren sie aus den verwendeten Lösungsmitteln ohne Schwierigkeiten aus.

**Beispiel 1**

o-Acetaminomethylbenzylmethylether

604 g (4 mol) o-Aminomethylbenzylmethylether wurden in 1500 ml Toluol gelöst und unter Rühren und Kühlen 408 g (4 mol) Acetanhydrid so zugesetzt, daß eine Temperatur von 60°C nicht überschritten wurde. Es wurde nachgerührt, mit 424 g 25 %iger Natronlauge neutralisiert und die wäßrige Phase abgetrennt. Beim Eindampfen der organischen Phase kristallisierte der acylierte Benzylether in einer Menge von 656 g, entsprechend 85 % der berechneten Menge aus. F. 67-68°C.

o-Acetaminomethylbenzylchlorid

Die erhaltenen 656 g (3,4 mol) Benzylether wurden bei 60°C wieder in 1500 ml Toluol gelöst und mit 1800 ml konzentrierter Salzsäure 5 h gerührt. Darauf wurden 1350 ml 25 %ige Natronlauge so zudosiert, daß 60°C nicht überschritten wurden. Nach Abtrennen der wäßrigen Phase setzte man zur organischen Phase 25 %ige Natronlauge zu, trennte die wäßrige wiederum ab und ließ die Chlorverbindung auskristallisieren. Ausbeute: 550 g, d.h. 82 % der berechneten Menge, bezogen auf den Benzylether. F. 69-70°C.

**Beispiel 2**

Es wurde wie in Beispiel 1 verfahren, jedoch wurde die Lösung des o-Acetaminomethylbenzylmethylethers in Toluol unmittelbar mit Salzsäure umgesetzt. Man erhielt in diesem Falle 571 g, d.h. 85 % der berechneten Menge. F: 68 bis 69°C.

**Patentansprüche**

1. Verfahren zur Herstellung von o-Acylaminomethylbenzylhalogeniden der allgemeinen Formel (I)

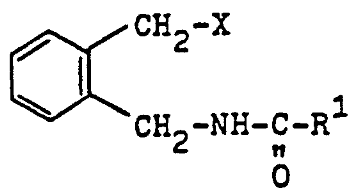

(I),

wobei X für ein Halogenatom und $R^1$ für einen aliphatischen oder aromatischen Rest steht, dadurch gekennzeichnet, daß man einen o-Aminomethylbenzylalkylether der allgemeinen Formel (II)

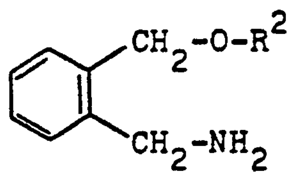

(II),

in der $R_2$ für einen Alkylrest steht, mit einem Acylierungsmittel zum entsprechenden Acylaminomethylbenzylalkylether und diesen bei einer Temperatur von bis zu 80°C mittels wäßriger Halogenwasserstoffsäure zum Halogenid (I) umsetzt.

2. Verfahren nach Anspruch 1, dadurch gekennzeichnet, daß man die Umsetzung mit Halogenwasserstoffsäure in Gegenwart eines mit Wasser nicht unbegrenzt mischbaren, den freien Halogenwasserstoff lösenden Lösungsmittels vornimmt.

**Claims**

1. A process for the preparation of an o-acylaminomethylbenzyl halide of the formula (I)

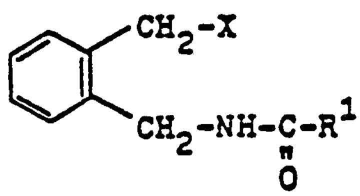

(I),

where X is halogen and $R^1$ is an aliphatic or aromatic radical, wherein an o-aminomethylbenzyl alkyl ether of the formula (II)

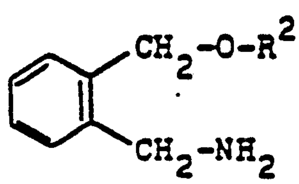

(II),

where $R^2$ is alkyl, is reacted with an acylating agent to give the corresponding acylaminomethylbenzyl alkyl ether, and the latter is reacted with an aqueous hydrohalic acid to give the halide (I).

2. A process as claimed in claim 1, wherein the reaction with the hydrohalic acid is carried out in the presence of a solvent which has a limited miscibility with water and dissolves the free hydrogen halide.

**Revendications**

1. Procédé de préparation d'halogénures de o-acylaminométhylbenzyle de la formule générale (I)

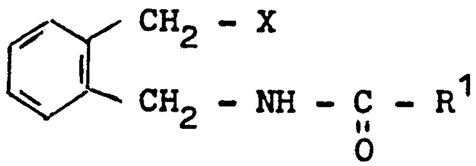

(I),

dans laquelle $R^1$ désigne un groupe aliphatique ou aromatique et X un atome d'halogène, caractérisé en ce que l'on fait réagir un éther d'alkyle du o-aminométhylbenzyle de la formule générale (II)

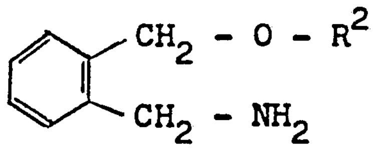

(II),

dans laquelle $R^2$ désigne un radical alkyle, avec un agent alkylant, puis l'on transforme l'éther d'alkyle de l'acylaminométhylbenzyle correspondant obtenu à une température pouvant aller jusqu'à 80° C, à l'aide d'acide halohydrique aqueux, en halogénure de la formule (I).

2. Procédé suivant la revendication 1, caractérisé en ce que la réaction avec l'acide halohydrique est réalisée en présence d'un solvant, qui dissout l'hydrogène halogéné libre, mais n'est pas miscible de façon illimitée à l'eau.